# EUROPEAN PATENT APPLICATION

(11) **EP 4 810 955 A1**
(43) Date of publication of application: **23.09.2026**
(21) Application number: 25165257.4
(22) Date of filing: 21.03.2025
(51) Int. Cl.: G01N 33/487, B01L 3/00

(54) **NANOPORE DEVICE, SYSTEM AND METHOD USING PINNED DROPLETS**

(71) Applicant: NMI NATURWISSENSCHAFTLICHES UND MEDIZINISCHES INSTITUT AN DER UNIVERSITÄT TÜBINGEN, 72770 Reutlingen (DE); Albert-Ludwigs-Universität Freiburg, 79098 Freiburg (DE); Ionera Technologies GmbH, 79108 Freiburg (DE)
(72) Inventor: Peter, Jones, 72144 Dusslingen (DE); Michael, Mierzejewski, 72127 Kusterdingen (DE); Jan, Behrends, 79252 Stegen (DE); Gerhard, Baaken, 79115 Freiburg (DE)
(74) Representative: Witte, Weller & Partner Patentanwälte mbB

(57) **Abstract**

The present invention relates to a nanopore device (10), in particular a nanopore array device, comprising: a substrate (30); one or more nanopores (20) in said substrate; a first fluid compartment (40) arranged at a first side of the substrate, wherein the first fluid compartment is fluidically coupled to the one more nanopores (20) from the first side; a first electrode (61) adapted to be electrically coupled to the first fluid compartment (40); wherein the nanopore device (10) is configured for formation of a pinned droplet (71, 72, 73) at at least one of said one or more nanopores (20) at a second side of the substrate (30), wherein the second side of the substrate is opposite to the first side of the substrate; and one or more second electrodes (81, 82, 83) adapted to be electrically coupled to the one or more pinned droplets (71, 72, 73) at said one or more nanopores (20). The present invention further relates to a corresponding system (11) and method (100) for operating a nanopore device.

## Description

### FIELD OF THE INVENTION

The present invention relates to devices and methods for the establishment of electrical and fluidic contact between one or more nanopores and one or more electrodes that can be connected to the input of one or more amplifiers in a way that permits high-density integration and independent measurement of electrical current through one or more nanopores at high bandwidth and resolution for the detection, discrimination and analysis of single molecules, including but not limited to the sequencing of biopolymers such as polynucleotides and polypeptides and proteins. The present invention thus relates to a nanopore device, in particular a nanopore array device, as well as to a corresponding system and method using such a nanopore device.

### BACKGROUND OF THE INVENTION

Nanopores are apertures of molecular dimension in a layer of electrically insulating material that establish a connection from one side of said layer to the other. When both sides are brought into contact and the nanopore is filled with a medium or solution containing mobile particles, these particles will be able to cross the layer from one side to the other, exclusively passing through the nanopore. If these particles are charged, e.g. are ions formed by solvated salts (i.e. the medium is a solution of electrolytes), then any net movement of them, whether by diffusion (random walk) or due to a directional force will produce an electrical current. Typically, such currents can be set up and/or or manipulated using electrodes on either side to provide a voltage bias across the layer. The resulting electrophoretic and/or electrodiffusive current can be measured using an appropriate electronic circuit, e.g. a potentiostatic (voltage clamp) amplifier, provided that the electrodes are capable of sustaining direct current. Such electrodes are typically electrodes of the second kind, e.g. metallic redox electrodes such as Ag/AgCl-electrodes.

In this situation, nonconductive particles (e.g. polymers or other molecules) added to the solution may enter the nanopore by diffusion or electrophoresis or under the influence of an (electro)osmotic or other convective force. While inside the pore, these particles will transiently alter the electric resistance of the pore. Most often, this transient alteration will consist in an increase in resistance (resistive pulse) through reduction of the conductive volume of the pore. Under certain circumstances (low concentration of charge carriers in the bulk solution) it can also consist in an increase in conductance (conductive pulse), e.g. when a cloud of counter ions dragged by the obstructing particle increases the concentration of charge carriers in the pore. In both cases, the waveform of the resulting current change contains information that can be used to determine important properties of the obstructing particle, such as its overall size or physicochemical nature or, in the case of a polymer, the sequence of its constituent monomers.

Nanopores can be of biological origin (biological nanopores) or man-made (Kasianowicz, J.J., J.W.F. Robertson, E.R. Chan, J.E. Reiner, and V.M. Stanford. 2008. Nano-scopic Porous Sensors. Annu. Rev. Anal. Chem. 1:737-766. doi:10.1146/annurev.anchem.1.031207.112818. // Dekker, C. 2007. Solid-state nanopores. Nature Nanotechnology. 2:209-215. doi:10.1038/Nnano.2007.27). Biological nanopores are typically proteins or complexes of proteins (pore-forming proteins, PFPs) or peptides that form aqueous channels through cellular membranes (Kasianowicz, J.J., A.K. Balijepalli, J. Ettedgui, J.H. Forstater, H. Wang, H. Zhang, and J.W.F. Robertson. 2016. Analytical applications for pore-forming proteins. Biochimica et Biophysica Acta (BBA) - Biomembranes. 1858:593-606. doi:10.1016/j.bbamem.2015.09.023). The biological role of the class of PFPs called porins is to serve the cellular metabolism as conduits for nutrients and waste or for transport of biopolymers such as proteins and nucleic acids. Other PFPs are released as pore-forming toxins (PFTs) by pathogens to permeabilize host cell membranes in order to bring about cell lysis (e.g. hemoly-sins). PFPs can be inserted into non-conducting soft-matter substrates, such as lipid or polymer membranes using well-established methods of reconstitution. While they can also be constructed from biomaterials using biomimetic self-organization, e.g. DNA-origami, or by pulling heated glass tubes to form apertures of sub-100 nm diameters, most artificial, man-made nanopores are typically produced using various nanoscale ablation or etching procedures applied to thin layers of solid-state materials such as silicon oxide, silicon nitride, graphene, molybdenum sulfate, boron nitride etc. To the person trained in the art these are known as solid-state nanopores. The insulating layers in which they are formed and which have thicknesses of no more than a few tens of nanometers are known as solid-state membranes.

Typically, fabrication procedures for solid-state nanopores use a focused beam of electrons or ions to form pores by local ablation and then optionally to reduce their diameter using lower energy/defocused beams (Li, J., D. Stein, C. McMullan, D. Branton, M.J. Aziz, and J.A. Golovchenko. 2001. Ion-beam sculpting at nanometre length scales. Nature. 412:166-169. doi:10.1038/35084037.; Kim, M.J., M. Wanunu, D.C. Bell, and A. Meller. 2006. Rapid Fabrication of Uniformly Sized Nanopores and Nanopore Arrays for Parallel DNA Analysis. Adv. Mater. 18:3149-3153. doi:10.1002/adma.200601191.; Merchant, C.A., K. Healy, M. Wanunu, V. Ray, N. Peterman, J. Bartel, M.D. Fischbein, K. Venta, Z. Luo, A.T.C. Johnson, and M. Drndić. 2010. DNA Translocation through Graphene Nanopores. Nano Lett. 10:2915-2921. doi:10.1021/nl101046t.Yang, J., D.C. Ferranti, L.A. Stern, C.A. Sanford, J. Huang, Z. Ren, L.-C. Qin, and A.R. Hall. 2011. Rapid and precise scanning helium ion microscope milling of solid-state nanopores for biomolecule detection. Nanotechnology. 22:285310. doi:10.1088/0957-4484/22/28/285310). Pore formation using a focused electron beam is a slow, serial process that cannot easily be translated into array formats. Pore formation using a focused ion beam can be automated for array formats, however it is also a serial process with limited scalability.

However, it would be desirable to provide large arrays of hundreds or thousands of nanopores in order for solid-state nanopores to be usable for meaningful analytical tasks, e.g. in high-throughput assays.

More recently, solid-state nanopores have also been formed by electron-beam lithography combined with chemical dry etching or reactive ion etching (Verschueren, D.V., W. Yang, and C. Dekker. 2018. Lithography-based fabrication of nanopore arrays in freestanding SiN and graphene membranes. Nanotechnology. 29:145302. doi:10.1088/1361-6528/aaabce). With his technique pores measuring several tens of nanometers can currently be formed but optimization of the method may enable pores of less than 10 nm in diameter to be formed in the future.

All of the above methods require vacuum and certainly cannot be performed while the membrane in which the nanopore is to be introduced is immersed in aqueous electrolyte solution. This is in contrast to biological nanopores which form *in situ* by a process of self-assembly controlled by the PFP structure that is not disturbed by the presence of e.g. salt and water or other matter. Therefore, to set up an array of biological nanopores, it is sufficient to provide an array of electrodes separated from a common reference or ground electrode by electrically insulating membranes in contact with solutions containing charge carriers. The array of pores is then formed in situ after pore forming protein is added to one side of the membrane in suitable concentration. Such a process, is, for instance shown in Baaken, G., M. Sondermann, C. Schlemmer, J. Rühe, and J.C. Behrends. 2008. Planar microelectrode-cavity array for high-resolution and parallel electrical recording of membrane ionic currents. Lab Chip. 8:938-944. doi:10.1039/b800431e. and Baaken, G., I. Halimeh, L. Bacri, J. Pelta, A. Oukhaled, and J.C. Behrends. 2015. High-Resolution Size-Discrimination of Single Nonionic Synthetic Polymers with a Highly Charged Biological Nanopore. ACS Nano. 9:6443-6449. doi:10.1021/acsnano.5b02096. Further documents show various means of automating the production of the electrically insulating membrane: US2013/0140192 A1 / del Rio Martinez, J.M., E. Zaitseva, S. Petersen, G. Baaken, and J.C. Behrends. 2015. Automated Formation of Lipid Membrane Microarrays for Ionic Single-Molecule Sensing with Protein Nanopores. Small. 11:119-125. doi:10.1002/smll.201402016.

A method that allows *in situ* formation of nanopores in solid-state materials such as silicon nitride is known as controlled dielectric breakdown (CDB) (Kwok, H., K. Briggs, and V. Tabard-Cossa. 2014. Nanopore Fabrication by Controlled Dielectric Breakdown. PLoS ONE. 9. doi:10.1371/journal.pone.0092880./). Here, a strong electric field is imposed on a solid-state membrane already immersed in electrolyte, as for recording. By processes not fully understood, the high field stochastically generates defects (charge traps) that can accumulate to create a percolation path for current that locally damages the membrane material (Briggs, K., M. Charron, H. Kwok, T. Le, S. Chahal, J. Bustamante, M. Waugh, and V. Tabard-Cossa. 2015. Kinetics of nanopore fabrication during controlled breakdown of dielectric membranes in solution. Nanotechnology. 26:084004. doi:10.1088/0957-4484/26/8/084004. / Fried, J.P., J.L. Swett, B.P. Nadappuram, J.A. Mol, J.B. Edel, A.P. Ivanov, and J.R. Yates. 2021. In situ solid-state nanopore fabrication. 1-20. doi:10.1039/D0CS00924E). The pore formation process can be followed by monitoring the ionic conductance through the pore and pore size can be controlled by discontinuing the electric field when a desired conductance value is reached. CDB, while attractive as a means of forming pores in situ, suffers from the unpredictability of both the time and the place of pore formation inherent in a stochastic process. In fact, even formation of a single pore, as required for sensing, is not guaranteed. Furthermore, while conceivable, parallel formation of nanopores in an array format by CDB has not been established for large scale parallelization, likely due to the difficulty of simultaneously monitoring pore conductance and controlling the voltage at many sites in parallel. A variant of the method, laser-assisted CDB, allows some control of the position at which the pore will be formed but is not easily amenable to parallel formation of pores in arrays.

### SUMMARY OF THE INVENTION

The inventors recognized that, at present, formation of solid-state nanopore arrays will therefore have to rely on fabrication procedures which do not tolerate the presence of electrolyte solution on either side of the membrane. The inventors further recognized that, consequently, following formation of a nanopore array, an important technical problem may consist in finding a way of contacting and filling each nanopore in said array with electrolyte solution and contacting said solution with electrodes of the second kind so that at least on one side the nanopore is in contact with an electrolyte-filled compartment that, in turn, is electrically contacted by a single or at least one electrode leading to a single or individual input stage of a potentiostatic (voltage clamp) amplifier, while on the other side all nanopores of said array typically may be in contact with the same electrolyte-filled compartment which in turn is electrically contacted by at least one electrode of the second kind serving as a common reference electrode.

The inventors recognized that, for successful independent recording from each nanopore in an array it would be desirable to have negligible resistive and capacitive coupling between the several electrolyte-filled compartments and between the several electrodes contacting said compartments and leading to the several input stages of the several potentiostatic amplifiers.

The inventors further recognized that it would be of great advantage if negligible resistive and capacitive coupling between said electrodes and compartments and amplifier input stages could be achieved despite a small distance between nanopores in an array (i.e. a small pitch) to enable high density of integration and, therefore, high throughput in nanopore sensing.

The inventors further recognized that it would further be highly advantageous for the total surface area of dielectric material covered by electrolyte solution in contact with each of the several electrodes leading to the several input stages of the several potentiostatic amplifiers to be as small as possible in order to minimize the capacitive load on the input stage. In voltage clamp experiments using a potentiostatic amplifier, the total capacitance of all surfaces in contact with the input stage is a major determinant of current noise because any random voltage fluctuations originating in e.g. the electronic components of the amplifier are reflected in this capacitance as current noise (Sigworth, F.J., and K.G. Klemic. 2005. Microchip Technology in Ion-Channel Research. IEEE Trans. NanoBiosci. 4:121-127. doi:10.1109/TNB.2004.842471).

It is an object of the present invention to provide a further improved nanopore device, system and method. In particular, it would be desirable to provide a nanopore device, system and method that fulfil one or more of the requirements cited above in order to provide a more compact device for parallel measurements with high throughput and high signal to noise ratio.

In a first aspect of the present invention, a nanopore device is presented that comprises: a substrate, in particular with a solid-state membrane; one or more nanopores in said substrate, in particular in said solid-state membrane; a first fluid compartment arranged on a first side of the substrate, in particular of the solid-state membrane, wherein the first fluid compartment is fluidically coupled to the one or more nanopores from the first side; a first electrode adapted to be electrically coupled to the first fluid compartment (in particular a first electrode adapted to be electrically coupled to a first fluid in the first fluid compartment); wherein the nanopore device is configured for formation of a pinned droplet at at least one of one or more nanopores at the second side of the substrate, wherein the second side of the substrate is opposite to the first side of the substrate; and one or more second electrodes adapted to be electrically coupled to the one or more pinned droplets at said one or more nanopores. Each individual pinned droplet can be contacting and filling a corresponding individual nanopore.

In a second aspect of the present invention, a system for performing nanopore-based analysis is presented, the system comprising: a nanopore device as disclosed herein; and an analyzer, wherein the analyzer is adapted to detect and determine physicochemical properties of one or more molecules, in particular of biological or synthetic polymers, in particular one or more of their size, shape and sequence, based on a current and/or voltage change over time across the one or more nanopores in said substrate acquired via the first electrode and the one or more second electrodes.

In a further aspect of the present invention, a method for operating a nanopore device, in particular a nanopore array device is presented, the method comprising the steps of: providing a nanopore device as disclosed herein; flushing the second side of the substrate with a first liquid, wherein part of the first liquid is retained between the one or more nanopores and the one or more second electrodes for formation of one or more pinned droplets; and flushing the second side of the substrate with a second fluid different from the first liquid for removing the first liquid around and/or between the one or more pinned droplets of the first liquid. In other words, a small fraction or respective part of the first liquid is retained at a respective individual nanopore for formation of a small volume pinned droplet as an individualized second fluid compartment providing a low capacitance.

Preferred embodiments of the invention are defined in the dependent claims. It shall be understood that the claimed nanopore array, system and method can have similar and/or identical preferred embodiments as the claimed device, in particular as defined in the dependent claims and as disclosed herein.

The present invention is based on the idea of using one or more pinned droplets as the one or more electrolyte-filled compartments in contact with the corresponding one or more electrodes leading to the one or more input stages of the one or more potentiostatic amplifiers. The side of the nanopore electrically connected to the input stage of an amplifier is here defined as the *trans*-side, with the corresponding fluidic compartment(s) being called the *trans*-compartment(s) while the opposite side, which is typically connected to the electrical ground is defined as the *cis*-side with the corresponding fluidic compartment(s) being called the cis-compartment(s). It is proposed to combine a nanopore (array) device, in particular a solid-state nanopore (array) device, with the formation of one or more pinned droplets, wherein each pinned droplet forms a fluidic and electrical connection between one trans-side (second) electrode out of one or more of such (second) electrodes and one nanopore out of one or more nanopores forming the nanopore array.

The inventors recognized that in preliminary experimental solid-state nanopore devices, sensor concepts are demonstrated with single nanopores, yet commercialization, in particular for fast and/or high throughput applications, will require parallel independent measurement at/from multiple nanopores. Measurement of a nanopore requires fluidic and electrical contact on both sides of the membrane (*cis* and *trans* compartments). If multiple nanopores are to be recorded from separately, either as individual sensors or in groups, these multiple nanopores may share a common *cis*-compartment, but there must be one separate *trans*-compartment in contact through an electrode with the input stage of each amplifier circuit used for recording. In other words, each amplifier input stage circuit used to record current through one element of an array of nanopores has to be electrically separated from all others and this also requires that the *trans* fluidic compartments in electrical contact with each circuit be separated from all other *trans* fluidic compartments. Note that an individual amplifier may be coupled to different second electrodes via a multiplexing circuit. Nonetheless, a plurality of amplifier circuits can advantageously be provided for parallelization.

The literature documents provide a limited number of attempts to produce a nanopore array device wherein a solid-state membrane with a plurality of pores is provided in a format intended for independent electrical recording from individual pores, but in separated individual fluidic channels or additionally using separate microfluidic valves using several individual electrodes and amplifier circuits (Tahvildari, R., E. Beamish, V. Tabard-Cossa, and M. Godin. 2015. Integrating nanopore sensors within microfluidic channel arrays using controlled breakdown. Lab Chip. 15:1407-1411. doi:10.1039/c4lc01366b.; Tahvildari, R., E. Beamish, K. Briggs, S. Chagnon-Lessard, A.N. Sohi, S. Han, B. Watts, V. Tabard-Cossa, and M. Godin. 2017. Manipulating Electrical and Fluidic Access in Integrated Nanopore-Microfluidic Arrays Using Microvalves. Small. 13:1602601. doi:10.1002/smll.201602601.; Fu, J., L. Wu, Y. Qiao, J. Tu, and Z. Lu. 2020. Microfluidic Systems Applied in Solid-State Nanopore Sensors. Micromachines (Basel). 11:332. doi:10.3390/mi11030332.; S. Zeng, C. Wen, S. -L. Zhang and Z. Zhang, 2020. A Nanopore Array of Individual Addressability Enabled by Integrating Microfluidics and a Multiplexer. IEEE Sensors Journal 20, 3,1558-1563, doi: 10.1109/JSEN.2019.2947713.; Rahman, M., M.J.N. Sampad, A. Hawkins, and H. Schmidt. 2021. Recent advances in integrated solid-state nanopore sensors. 1-23. doi:10.1039/D1LC00294E.; Jones, P.D. and M. Mierzejewski, "Integration of solid-state nanopore arrays via dry bonding to photostructured microfluidic networks", Journal of Micromechanics and Microengineering, Vol. 35, No, 4, 2025]). In these approaches, one side of a membrane forms the shared cis-compartment. Separate trans-compartments are formed by multiple microfluidic channels on the opposite side of the membrane. Each microfluidic channel provides a separate trans compartment contacting a single nanopore. A main problem of this solution is that each microfluidic channel requires its own inlet and outlet. Handling such systems can be impractical. Moreover, miniaturization is substantially limited. In addition, *trans*-compartments consisting of microfluidic channels filled with conducting solution may lead to increased noise due to increased capacitive load and facilitate pickup of environmental electrical disturbances (e.g. power grid frequency hum) by acting as antennas. An alternative approach could be providing pre-filled compartments, wherein the compartments are pre-filled and then a membrane for the nanopores over the respective compartments is formed afterwards. However, such an approach of providing pre-filled compartments may not be desirable for solid-state nanopores with permanent membranes. Hence, the choice of materials for membranes may be limited and the fabrication effort may be substantial.

The inventors thus propose a novel approach in the specific field of nanopore devices of combining a nanopore device with the formation of one or more pinned droplets at corresponding pores, wherein each of pinned droplet can serve as a *trans* compartment according to the definition above in a nanopore device. The formation of pinned droplets is for example described in Theodorakis PE, et al. "Droplet Control Based on Pinning and Substrate Wettability", Langmuir. 2021 Apr 13;37(14):4248-4255. doi: 10.1021/acs.langmuir.1c00215 as well as in Feng et al., "Droplet Microarrays: From Surface Patterning to High-Throughput Applications", Advances Materials, Vol 30, Issue 20, 2018, doi.org/10.1002/adma.201706111, the contents of which are incorporated herein by reference. The proposed nanopore device comprises a substrate and one or more pores or nanopores in said substrate. The substrate or portions thereof can be configured as a membrane for a nanopore measurement with respective one or more openings referred to as nanopores. On a first side of the substrate, a first fluid compartment is provided. The first fluid compartment is fluidically coupled to the one or more nanopores from the first side of the substrate. The first fluid compartment can serve as a *cis* or *trans* compartment according to the definition above. The first electrode is adapted to be electrically coupled to the first fluid compartment. Hence, a first electrode is provided for establishing an electric contact to the fluid in the first fluid compartment during operation. The first electrode can for example be provided on the first side of the substrate or may be a discrete electrode in contact with the fluid compartment.

The substrate with the nanopores can be a prefabricated substrate. In particular, the substrate can be a solid-state substrate. The nanopore can be a solid-state nanopore. The substrate can be provided as or can comprise one or more solid-state membranes, in particular a solid-state membrane which cannot be formed *in situ.*

An advantage of solid-state substrates is that they can be prefabricated with high accuracy. A further advantage is that a solid-state substrate and solid-state nanopores can provide a high chemical stability. In particular, a solid-state nanopore can be more robust than a purely biological nanopore. For example, the substrate can be a silicon (Si) substrate e.g. made from a Si wafer with 200 µm thickness. The substrate can comprise one or more layers. For example, a silicon nitride (SiN) layer can be provided on the silicon wafer. The silicon nitride layer can have a thickness of 1 nm to 100 nm, in particular of 10-100 nm, for example at thickness of 3 nm. It is to be understood that Si and/or SiN membranes are here mentioned as non-exhaustive examples and a membrane of another suitable material may be provided. It is also possible to provide a SiN membrane in combination with one or more layers of other materials. The materials may be atomically thin, e.g. graphene or other 2D materials such as hBN or MoS2. The diameter of the nanopore may vary along its length, e.g. with a wide region in SiN and a narrow region in a 2D material. A length may refer to a length in depth or thickness direction perpendicular to the layers. For example, a 100 nm thick SiN membrane may be provided with a 100 nm aperture, covered by a layer of e.g. a 2D material with an aperture of smaller diameter forming a nanopore, for example a 5 nm nanopore. It is also possible that nanopores may be coated with another material, e.g. HfO2 or Al2O3 by ALD (atomic layer deposition) after nanopore formation. An array of nanopores can be provided in the substrate. The nanopores can have a width or diameter of e.g. between 1 nm and 100 nm, in particular between 3 nm and 50 nm. A further advantage of a nanopore device having a solid-state substrate and/or solid-state nanopore can be that the shelf-life of the device can be increased. This can reduce inventory costs and avoid incorrect measurements due to expired devices.

The proposed nanopore device is configured for formation of a respective pinned droplet at at least one of said one or more pores in the substrate at a second side of the substrate. If a plurality of nanopores is provided, the proposed nanopore device can be configured for formation of a respective separated pinned droplet at at least some of said plurality of nanopores. Each of said pinned droplets can form a (sub-) compartment for the nanopore measurement. One or more second electrodes, in particular an array of second electrodes is adapted to be electrically coupled to the one or more pinned droplets at (at least some of) said one or more pores. Hence, a nanopore measurement across the pore between cis and trans compartments can be executed for one or more individual pores of the nanopore device.

At first glance, it may seem counterintuitive to use pinned droplets for formation of a *cis* or *trans* compartment in a nanopore device. There may indeed be some statistical uncertainties and the probability that a pinned droplet actually forms may not be 100%. Nonetheless the inventors recognized that, in particular when providing an array for formation of pinned droplets, such statistical uncertainties and failure to form a droplet at a few pores may be tolerated. An advantage can be that the use of pinned droplets allows to substantially increase the number and/or density of nanopores by miniaturizing the fluid connection between each of the several electrodes and its corresponding nanopore outweighs that risk.

A further advantage of providing a nanopore device configured for formation of one or more pinned droplets at (at least some of) said one or more nanopores is that the respective fluid compartment or sub-compartment can be provided by a pinned droplet. A pinned droplet typically provides a very low fluid volume per nanopore. This allows the user to work with very low volumes of the material to be analyzed. Such material may be precious and/or may only be available in limited quantities. For example compounds or cells from which only a very limited amount is available or that would have to be cultivated at high cost. Quantities as low as a few hundred nanoliters can be sufficient.

A further advantage can be that a very compact device can be provided. An array of nanopores configured for formation of pinned droplets may be provided with a high density of nanopores per unit area. In particular, it may no longer be necessary to provide separate fluidic channels with separate inlets and outlets for each individual nanopore. In the prior art (see references cited above), the number of separate fluidic channels with inlets and outlets is a limiting factor for the number of nanopores in an array.

A further advantage can thus be that the number of nanopores and thus the number parallel measurements or parallel measurement channels can be increased.

A further advantage can be that the spatial arrangement or density of measurement sites can be further improved. In view of the small size of pinned droplets, the distance between neighboring nanopores may be reduced.

A further advantage can be that the manufacturing cost can be reduced. In particular, the structural complexity of a nanopore device can be reduced.

A further advantage can be that the usable frequency bandwidth can be increased. Due to its low sample volume, a pinned droplet provides a low capacitance. By enabling a small distance between a nanopore and its respective second electrode, the droplet provides low resistance. This reduces capacitive noise and allows low-pass filtering with higher cut-off frequencies. At the same time, the time-constant of the RC-electrical circuit formed by the pore, substrate and fluid in contact with the amplifier input stage is reduced. With low capacitance or higher bandwidth, shorter resistive or conductive pulses, typically caused by smaller, more mobile or more highly charged analyte molecules, can be resolved. It may also be possible that a molecule to be sampled can be passed at a higher velocity through the nanopore, thereby enabling a higher rate of sampling of subsequent molecules passing through a nanopore. In other words, a higher number of molecules may be analyzed per unit time so that the throughput can be increased.

In the following, some terms which are used throughout the application, shall be shortly explained and defined:

As used herein, a pinned droplet can refer to a droplet of liquid that is immobilized or fixed at a specific location on a surface, usually due to surface tension forces or interaction with surface features, surface structures or topographical features. The proposed nanopore device is adapted such that respective pinned droplets form at at least some of said one or more pores at the second side of the substrate. The device can thus be configured such that a first pinned droplet forms at a first nanopore, a second pinned droplet forms at a second nanopore and so on. It is noted that a pinned droplet may form at all or only some nanopores of the device.

A pinned droplet as used herein can refer to a quantity of liquid which maintains a certain shape and/or position due to surface tension, contact to solid surfaces, and the presence of other fluids. The liquid can be conductive, for example, water containing dissolved salts (dissociated ions). Other solvents and other conductive fluids such as ionic liquids may also be used. The nanopore device can be adapted for immobilizing the respective one or more droplets at the location of a respective nanopore by features such as surface energy, hydrophilicity/hydrophobicity, roughness, chemical heterogeneity, or topography. The droplet can be in contact with the substrate, surrounded by insulating solid surfaces and other fluids, which may be liquids or gases. Pinned droplets typically occur when the contact line of the droplet (the boundary where the liquid, solid, and a second fluid e.g. air meet) is anchored to surface imperfections, physicochemical heterogeneities, or micro/nanostructures on the surface. These features or imperfections can create areas of varying wettability, causing the droplet to become pinned.

As used herein, a pore or nanopore can refer to a typically nanometer-sized hole or aperture in the substrate. Pores or apertures may be purely solid-state or may host a biological protein or may otherwise be chemically or biologically functionalized. In the context of a nanopore device, the substrate with the one or more pores may also be referred to as a membrane that separates the one or more cis and one or more trans compartments on the opposite sides of the substrate. The substrate can be an insulating substrate. Advantageously a solid-state substrate is provided. The first and/or second electrode can be provided on the substrate on the respective sides to contact the respective compartments. An electrode can for example be a silver (Ag), silver-chloride (AgCl), gold (Au) electrode. In particular, a non-redox electrode may be provided. Redox couples may be provided in the liquid. An electrode can be provided by different manufacturing methods, including but not limited to microelectrochemical deposition or vapor deposition.

As indicated above, the nanopore device can be a nanopore array device. The nanopore array device can comprise a plurality of nanopores in said substrate; wherein the first fluid compartment is fluidically coupled to the plurality of nanopores from the first side; wherein the nanopore array device is configured for formation of a pinned droplet at at least some of said plurality of nanopores at the second side of the substrate; and an array of second electrodes adapted to be electrically coupled to the pinned droplets at at least some of said plurality of nanopores.

For formation of the one or more pinned droplets at the respective one or more nanopores, at each of said one or more nanopores there can be provided a confinement structure on the second side of the substrate. In other words, there can be provided a confinement structure near each of said pores on the second side of the substrate. A confinement structure may be on the surface of the second side of the substrate. A confinement structure may otherwise be separated from the substrate, but near enough that a pinned droplet formed at the confinement structures makes contact to the substrate and thereby to a nanopore. For example, the device can be adapted for immobilizing a droplet at the location of a nanopore by properties such as surface energy, roughness, chemical heterogeneity, or topography. The droplet thus forms at a specific location, depending on specific structures and/or wettability adapted for formation of a droplet. Such a confinement structure or set of local surface properties can also be referred to as a droplet trap adapted to retain a droplet at a respective pore. The retained droplet can provide a fluid compartment, such as a *cis* or *trans* compartment, for a nanopore measurement at said pore. It will be understood that the formation of a pinned droplet can thus be achieved in different ways such as by different microstructures and/or optionally regional or local patterns of different surface energy/contact angle. The respective features may be selected depending on the fluid used for formation of the pinned droplets.

The confinement structure can be adapted to capture and retain a droplet from a fluid passing said confinement structure on the second side of the substrate. An advantage of this embodiment is that the respective fluid for the compartment to be provided by the droplet can be provided very efficiently. A further advantage is that a very low sample volume can be used. This is particularly advantageous when working with precious samples or samples with low availability.

The nanopore device can be configured such that the second side of the substrate at the nanopore is accessible. The nanopore device can be configured such that a droplet at the nanopore is exchangeable by passing a second fluid at the second side of the substrate for formation of a second droplet of the second fluid at the nanopore. The second fluid can be a same or different fluid than the fluid for formation of a first fluid for formation of a first droplet.

Each respective confinement structure at each respective pore can be provided by a region having a different wettability and/or surface energy and configured such that a pinned droplet is formed and retained at said region having the different wettability and/or surface energy.

In an embodiment, the nanopore device can be adapted for formation of one or more pinned droplets each having a fluid volume of at most 100 nL (nano-liters), in particular having a fluid volume between 5 pL (pico-liters) and 100 nL, in particular between 7 pL and 50 nL, in particular between 10 pL and 1 nL, in particular between 5 pL and 100 pL. For example, a confinement structure can be adapted accordingly. Advantages can include one or more of a low fluid volume, low capacitance, low resistance, high useable bandwidth and high rate of sampling of analyte molecules.

The nanopore device can be configured for formation of one or more pinned droplets having a diameter between 1 µm and 1 mm, in particular between 10 µm and 700 µm, in particular between 20 µm and 200 µm. For example, a confinement structure can be adapted accordingly. Thereby, a very compact device or nanopore array with a high density of nanopores may be provided.

The nanopore device can be configured for formation of one or more pinned droplets having a height of at most 250 µm, in particular having a height between 5 µm and 200 µm, in particular between 7 µm and 150 µm, in particular between 10 µm and 100 µm. For example, a confinement structure can be adapted accordingly. Advantages can include one or more of a low fluid volume, low capacitance, low resistance, high useable bandwidth and high rate of sampling of analyte molecules.

For example, the device may be adapted for formation of individual droplets at one or more of the pores having a volume of about 20 to 40 pL, having a diameter of about 50 µm, a height of about 10 to 20 µm. In a further example, the device may be adapted for formation of individual droplets at one or more of the pores having a volume of about 5 pL, a diameter of about 30 µm and a height of about 7 µm. In a further example, the device may be adapted for formation of individual droplets having a volume of up to 100 nL, a diameter of no more than 1 mm and a height of no more than 125 µm.

A pitch between neighboring nanopores of the nanopore array device can be between 50 µm and 1.5 mm, in particular between 100 µm and 1 mm, in particular between 0.2 mm and 0.7 mm. As used herein, the pitch can refer to the center-to-center distance. An advantage of a pinned droplet array is thus that a nanopore array device with high density can be provided. A high density also enables a low sample volume to be used and is thus well suited for expensive analytes. A further advantage is that a high level of parallelization is possible.

In one embodiment, at least two nanopores can be fluidically coupled on the second side of the substrate. In particular, the nanopores can be fluidically coupled on the second side of the substrate where the device is configured for formation of the pinned droplets. Hence, the droplets can be fluidically coupled e.g. during formation of the droplets by flushing the second side of the substrate for formation of the pinned droplets. The fluidic coupling can also be used for exchanging a fluid for formation of the pinned droplets. An advantage can be that several subsequent measurements can be performed with the same device in an efficient manner. Despite being fluidically coupled, droplet formation results in electrical insulation between droplets.

In a refinement, a fluidic channel connecting at least some of the nanopores can be provided on the second side of the substrate. Hereby, the droplets can be formed in a microfluidic channel. An advantage of providing a fluidic channel connecting at least some of the pores is that the fluid for formation of the droplets can be efficiently provided to multiple pores. A further advantage is that the channel can be shared thereby reducing the device size. In other words, it is no longer necessary to provide a separate channel with a separate inlet and outlet for each separate pore. As an alternative to providing a fluid channel, the nanopore device may be adapted for formation of the one or more pinned droplets at at least some of the one or more nanopores of at an open second side of the substrate, for example by dipping the device into a liquid for the pinned droplets.

In addition or in the alternative, the nanopores can also be fluidically coupled on the first side of the substrate. In other words, at least some of the pores can be fluidically coupled on both sides of the substrate. For example, a common cis compartment can be provided on the first side of the substrate. It should be noted that fluidic coupling can automatically provide electrical coupling unless an electrically insulating fluid is included, for example in a two-phase fluidic system with a first electrically conductive liquid and a second electrically insulating fluid.

The nanopore device can be configured for providing the one or more pinned droplets by flushing the second side of the substrate with a first liquid, wherein part of the first liquid is retained at the one or more nanopores for formation of the pinned droplets. The second side of the substrate can be flushed with a second fluid different from the first liquid for removing the first liquid from around or between the one or more pinned droplets of the first liquid. The second side of the substrate can then be flushed by a third liquid for formation of new pinned droplets of the third liquid. This process can be repeated for formation of further pinned droplets of different liquids and/or subsequent measurements. As used herein, flushing can also include dipping. Hence, the second side of the substrate may also be dipped into the first liquid for formation of the droplets and the first liquid may be removed between the droplets by exposing the nanopore device to a second fluid. The second fluid can be a liquid or can also be a gas such as e.g. air. For example, a single fluidic channel connecting all pores can be flushed with a first liquid for formation of the pinned droplets. The droplets can then be formed and isolated from each other by using a second fluid (air/oil/etc.). The second fluid can be a non-conductive fluid for isolation. Thereby, a "one-to-many" configuration having one microfluidic channel for many nanopores can be provided.

The nanopore device can be further configured for formation of a pinned droplet at said one or more nanopores at the first side of the substrate. Optionally, both the *cis* and the *trans* compartment can be formed by pinned droplets. An advantage of this approach is that a sample volume can be further reduced. Hence, the first fluid-compartment can be provided as a plurality of first sub-compartments. A plurality of first electrodes coupled to the respective first fluid compartments can be provided.

In an embodiment, at least some of the one or more nanopores can be chemically or biologically functionalized, in particular wherein one or more nanopores host a lipid bilayer or polymer membrane, and in particular wherein one or more of the nanopores host a biological protein. This may also be referred to as a hybrid nanopore. An advantage can be that further measurements can be performed.

The nanopore device can be a non-prefilled device. In other words, the nanopore device can be provided without the first fluid compartment and/or without the one or more pinned droplets being prefilled with the liquid required for a desired measurement task. It will be appreciated that a placeholder liquid, different from a liquid for performing the nanopore measurement, may nevertheless be provided for shipping. For example, the nanopore device can be prefilled with a non-conductive liquid or gas, in particular oil. This can prevent undesired contamination. In other words, a non-prefilled device means that both the first fluid compartment as well as the second side of the substrate are fluidically accessible in the nanopore device for the user. An advantage of this embodiment is that the nanopore device can be used in a very flexible manner for different applications. Moreover, the shelf-time of the device can be increased. This can reduce inventory costs and avoid incorrect measurements due to expired devices.

The nanopore device can be a nanopore array device adapted to electrically isolate neighboring droplets due to separation by an electrically insulating fluid. For example, the device can be configured such that neighboring droplets can be electrically isolated from each other by a fluid such as air or a non-conductive liquid. The neighboring droplets may still be fluidically coupled. However, no electrical connection is provided in view of the electrically insulating fluid. Hence, instead of providing structural hardware barriers between neighboring droplets, the droplets are electrically isolated from each other by an electrically insulating fluid. An advantage can be a more cost-effective implementation, reduced manufacturing complexity and/or reduced handling complexity during use.

In an embodiment, a first group of droplets can share a first common *cis* or *trans* compartment and a second group of droplets can share a second common *cis* or *trans* compartment.

For each pore of the nanopore array, a separate second electrode may be provided. The electrodes may be addressed and read out individually. In particular when using a solid-state substrate, individual electrodes for the individual nanopores can be implemented with a high degree of miniaturization and in a very cost-effective manner.

### BRIEF DESCRIPTION OF THE DRAWINGS

These and other aspects of the invention will be apparent from and elucidated with reference to the embodiment(s) described hereinafter. In the following drawings
- Fig. 1: shows a schematic diagram of a single solid-state nanopore device with a dedicated fluid-filled channel per nanopore;
- Fig. 2: shows a schematic diagram of a first exemplary embodiment of a nanopore device according to an aspect of the invention;
- Fig. 3: shows a schematic top view of a second exemplary embodiment of a nanopore device;
- Fig. 4: shows a schematic cross-sectional view of the second exemplary embodiment of a nanopore device of Fig. 3;
- Fig. 5: shows a schematic perspective view of the second exemplary embodiment of a nanopore device of Fig. 3 and 4;
- Fig. 6: shows the process of flushing a nanopore device with a first and second fluid for formation of pinned droplets;
- Fig. 7: shows a schematic top view of a further exemplary embodiment of a nanopore device;
- Fig. 8: shows an isometric view of an embodiment of a nanopore device;
- Fig. 9: shows a schematic cross-sectional view of a further exemplary embodiment of a nanopore device;
- Fig. 10: shows the process of forming droplets on both sides of a further exemplary nanopore device;
- Fig. 11: shows a flow chart of a method according to an aspect of the present invention.

### DETAILED DESCRIPTION OF THE INVENTION

Commercial success has been achieved with biological nanopores, while promising basic research with solid-state nanopores remains to be translated into practical use. Parallelization has proven to be an important factor for efficient research on biological nanopores and is critical for practical use.

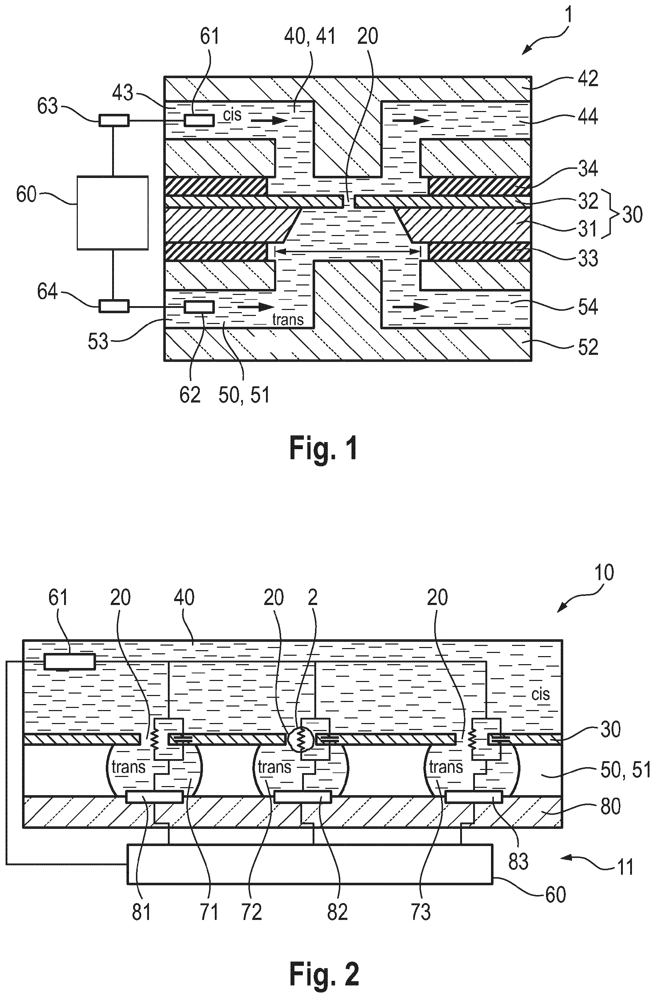

Fig. 1 shows a schematic diagram of a solid-state nanopore device 1 with a dedicated fluidic and fluid-filled channel per nanopore. In the example shown, a single nanopore 20 is provided in a substrate 30. The substrate 30 can be a solid-state substrate comprising for example a first support layer 31, in particular a silicon layer, on which a silicon nitride layer 32 is provided and acts as a membrane for formation of the actual nanopore 20. The substrate can comprise a thicker silicon layer 31 with an electrically insulating thin layer 32 of SiO2 and/or SiN or other materials, in particular an electrically insulating material. It can feature an area of some 10s of µm² where the silicon layer 31 is removed exposing the thin electrically insulating layer 32 from both sides. However, it may also be possible to provide a single-layer substrate or a substrate having further layers, for example for providing measurement electrodes and/or routing layers for routing electrical signals e.g. to a periphery.

A first fluid compartment 40 is arranged on a first side of the substrate 30, here on the top side. A second fluid compartment 50 is arranged on a second side of the substrate 30, here on the bottom side. The second side of the substrate is opposite to the first side of the substrate. The first and second fluid compartments 40, 50 provide the *trans* and *cis* compartments for the nanopore measurement. For example, the first fluid compartment 40 can serve as the *cis* compartment and the second fluid compartment 50 can serve as the *trans* compartment. However, the disclosure is not limited thereto. As described above, the side of the nanopore electrically connected to the input stage of an amplifier can be referred to as the trans-side, with the corresponding fluidic compartment(s) being called the trans-compartment(s) while the opposite side, which is typically connected to the electrical ground can be referred to as the cis-side with the corresponding fluidic compartment(s) being called the cis-compartment(s).

As shown in Fig. 1, the first fluid compartment 40 is provided by a first fluidic channel 41 being filled with a fluid for the nanopore measurement. The first fluidic channel 41 can for example be formed in a layer 42 such as a polycarbonate layer or other suitable material for formation of a fluidic channel. The first fluidic channel 41 is provided between an inlet 43 and an outlet 44. However, it is also possible to implement the first fluid compartment in different forms, such as an open chamber.

In the shown example, the second fluidic compartment 50 is provided by a second fluidic channel 51 being filled with a fluid for the nanopore measurement. For example, phosphate-buffered saline (PBS) or other suitable fluids can be used depending on a desired measurement. The second fluidic channel can for example be formed in a layer 52 such as a polycarbonate layer or other suitable material for formation of a fluidic channel. The second fluidic channel 51 is provided between an inlet 53 and an outlet 54. Accordingly, in the shown example, the second fluidic channel 50 only provides access to a single nanopore 20.

In the example shown in Fig. 1, optional layers 33 and 34 provide a gasket for sealing the area around the nanopore 20.

For performing the measurement across the nanopore, an analyzer 60 is provided. The nanopore device 10 and analyzer 60 together can be seen as a system 11 for performing nanopore-based analysis. The analyzer 60 is connected to a first electrode 61 via a first input terminal 63 and to a second electrode 62 via a second input terminal 64. The first electrode 61 is electrically coupled to the first fluid compartment 40 provided by the first fluidic channel 41 being filled with the fluid, e.g. the fluid of the cis compartment, for the nanopore measurement. The second electrode 62 is electrically coupled to the second fluid compartment 50 provided by the second fluidic channel 51 being filled with the fluid, e.g. the fluid of the trans compartment, for the nanopore measurement. The analyzer 60 can comprise an amplifier adapted to perform potentiostatic (voltage clamp) current measurements. For example, a commercially available patch clamp amplifier such as an Axopatch 200B from Molecular Devices, an EPC-8 from HEKA, a dPatch from Sutter Instruments or an E-one amplifier from Elements can be used. Alternatively, a so-called current clamp amplifier can be used to measure voltage instead of current signals. The analyzer can also comprise a suitable analog-to-digital converter in order to provide current or voltage signals that can be treated and stored in digital form.

In the example shown in Fig. 1, the first input terminal 63 may be coupled to electrical ground. The first fluid compartment can thus serve as the cis compartment. The second input terminal 64 may be coupled to the input stage of an amplifier and can thus serve as the trans compartment. For example, a suitable amplifier circuit with an operational amplifier may be coupled to the second input terminal 64.

Fig. 2 shows a schematic diagram of a first exemplary embodiment according to an aspect of the present invention of a nanopore device 10, here a nanopore array device with a plurality of nanopores 20 with associated pinned droplets 71, 72, 73. The nanopore array device 10 comprises a substrate 30 with a plurality of nanopores (pores) 20 in said membrane or substrate 30. The substrate 30 can comprise one or more layers as already described above. It shall be understood that, while the device 10 is exemplarily illustrated as a nanopore array device with a plurality of nanopores, the same considerations apply accordingly for a nanopore device with a single nanopore. In the following, reference will be made to a nanopore array device.

The substrate can for example be a silicon (Si) substrate e.g. made from a Si wafer with 200-500 µm thickness. The substrate can comprise a support layer with apertures. An additional SiN layer can be provided over the carrier with apertures in the SiN layer aligned with the apertures in the Si carrier layer. An advantage of the approach is that well-established large scale and low-cost semiconductor fabrication techniques with high precision may be used for manufacturing. This allows to achieve high quality devices with high manufacturing yield. The apertures in the SiN layer can typically have a smaller size than the apertures in the carrier layer. The apertures in the SiN layer can thereby define the openings of the nanopores. The pores of the nanopore array device can already be opened or can also be provided in a to-be-opened state or the membranes may be provided without nanopores, but with suitable properties for in situ nanopore formation. In other words, the pores can already be provided in the nanopore array device but in a state where they have to be opened, e.g. by wetting or etching, prior to using the nanopore device. Wetting or etching could require the application of chemicals, solvents, plasma or voltage. If the membranes are provided with nanopores, the nanopore may be formed in situ, e.g. by controlled dielectric breakdown, laser induced pore formation, or a combination. The membranes can have features such as electrodes or thinned regions to support in situ pore formation. Note that it can also be possible to form the pinned droplet prior to opening the nanopore. It will be appreciated that nanopores can be formed in different ways. It is also possible that the pores be provided in the nanopore array device but in a non-functional state where they have to be primed, e.g. wetted or provided with a bio-functional layer, prior to using the nanopore array device.

A first fluid compartment 40 is arranged on a first side of the substrate 30, here on the top side. The first fluid compartment 40 is fluidically coupled to the plurality of nanopores 20 from the first side. The first fluid compartment 40 can be provided in different forms, e.g. as an open fluid compartment such as an open well in a single or multi-well microtiter plate or can also be provided as a fluidic channel, as e.g. illustrated above in Fig. 1. A first electrode 61 is electrically coupled to the first fluid compartment 40.

A second fluid compartment 50 can be provided on a second side of the substrate, here on the bottom side. For example by a fluidic channel 51 formed between the substrate 30 and a carrier 80. The carrier can be a further substrate, for example a solid-state substrate or glass substrate known from microelectrode array technology. The second fluid compartment 50 is separated into two kinds of volumes, those occupied by pinned droplets consisting of conductive fluid, also referred to as sub-compartments, and those that are instead occupied by an electrically insulating fluid such as air or other non-conductive fluid. More precisely, the nanopore array device 10 according to aspects of the present disclosure is configured for formation of one or more respective pinned droplets 71, 72, 73 at at least some of said one or more pores 20 at a second side of the substrate, here at the bottom side of the substrate. The second side of the substrate with the respective pinned droplets thereby provides the respective other (sub-)compartments, e.g. of the *cis* and *trans* compartments, for parallel nanopore measurements. In other words, the plurality of pinned droplets 71, 72, 73 serve as a plurality of second fluid (sub-)compartments on the opposite side of the nanopores 20 of the substrate than the first fluid compartment 40. For example, the first fluid compartment 40 can be a common *cis* compartment and the pinned droplets 71, 72, 73 provide a plurality of separate *trans* compartments for separate nanopore measurements.

Note that a common first fluid (*cis*) compartment 40 may be provided or that a plurality of first fluid compartments or sub-compartments can be provided. Multiple first fluid compartments can be used to perform parallel measurements with different fluids or samples.

The nanopore device 10 further comprises one or more, in particular an array of, second electrodes 81, 82, 83 adapted to be electrically coupled to the pinned droplets at at least some of said one or more nanopores.

In the example shown in Fig. 2, the second electrodes 81, 82, 83 are provided on a separate carrier 80 that is arranged on the second side of the substrate 30. The separate carrier 80 can be a carrier as for example used in micro electrode arrays. This allows efficient manufacturing with well-established techniques that may also provide biocompatibility as required by a desired measurement application.

Alternatively, the second electrodes 81, 82, 83 can be provided directly on the second side of the substrate 30, in other words on the surface of the substrate 30 facing towards the second side. An advantage of this approach is efficient manufacturing. For example, a much simpler separate carrier 80 can be used if fluid compartment or fluidic channel 51 is desired between the substrate 30 and the carrier 80. Alternatively, it is also possible that the second side of the substrate 30 is open. The pinned droplets 71, 72, 73 can thus also form on an open second side of the substrate 30. An exemplary embodiment is also shown further below.

For performing the measurement across the plurality of nanopores, an analyzer 60 is provided. The analyzer 60 is adapted to measure one or more molecules based on a current and/or voltage change over time across the plurality of pores 20 in said substrate 30 acquired via the first electrode 61 and the array of second electrodes 81, 82, 83.

Accordingly the analyzer 60 is connected to a first electrode 61 and to the array of second electrodes 81, 82,83. The first electrode 61 is electrically coupled to the first fluid compartment 40 being filled with the fluid, e.g. the fluid of the cis compartment, for the nanopore measurement. In the example shown in Fig. 2, each second electrode 81, 82,83 is electrically coupled to a corresponding pinned droplet 71, 72, 73. Each pinned droplet provides a separate sub-compartment, e.g. a separate trans compartment, for separate nanopore measurements. It is thus possible to perform current and/or voltage recordings, for example using one or more adapted amplifiers, across a plurality of separate nanopores in a very efficient and compact manner. The first electrode may be coupled to ground. The analyzer 60 can comprise multiple channels for parallel measurements. However, it is also possible to provide a multiplexer. The multiplexer can be coupled to the second electrodes 81, 82, 83 and selectively couple the respective electrodes for measurement in the analyzer. Compared to a fully parallel measurement, this can reduce the system cost. It is also possible to provide a combined approach wherein a certain number of parallel measurement channels is provided and each measurement channel can be selectively connected to a plurality of second electrodes.

Fig. 2 further illustrates a simplified circuit diagram of the electrical characteristics of nanopore measurement. At each nanopore 20, a simplified circuit diagram section with a capacitor and a resistor is shown. A measurement object 2, such as a molecule or polymer including but not limited to DNA or RNA, located at or passing through the nanopore 20 alters the electrical characteristics and can thus lead to a measurement signal that can be acquired with the analyzer 60. An advantage of the proposed nanopore array device is that the use of a pinned droplet for at least one (sub-)compartment of the nanopore measurement may significantly reduce the capacitance and thereby enable high bandwidth and/or fast measurements. It shall be understood that a separate pinned droplet for each nanopore can be used. Compared to the filled fluidic channel 51 illustrated in Fig. 1, the pinned droplets 71, 72, 73 each provide a much smaller fluidic volume and thus a much smaller capacitance per nanopore. The capacitance may be determined by the surface area of the fluidic volume electrically contacted. Moreover, much less sample material is required for the measurement.

For formation of the pinned droplets 71, 72,73 at the respective pores 20 at each of said pores there is provided a confinement structure at the second side of the substrate 30. The confinement structure can be provided directly by the substrate. However, it is also possible that the confinement structure is provided by a separate carrier 80 at the second side of the substrate 30. This can facilitate fabrication. The confinement structure can be adapted to capture and retain a droplet from a fluid passing said confinement structure at the second side of the substrate 30. This can simplify formation of the pinned droplets 71, 72, 73. The confinement structure can be provided by a region having a different wettability and/or surface energy and configured such that a pinned droplet is formed and retained at said region having the different wettability and/or surface energy or by micro-/nanostructures. For example, such a region can be provided on the substrate 30 or on the separate carrier 80. It is also possible, that a confinement structure for a pinned droplet 71 is provided by a second electrode 81. Thereby, a synergy effect can be provided since the electrode serves both for the electrical measurement and as a confinement structure. The same can apply for the further electrodes.

The pores 20 are configured to be fluidically coupled on the second side of the substrate 30, in particular the pores are configured to be fluidically coupled on the second side of the substrate 30 where the device is configured for formation of the pinned droplets 71, 72, 73. This allows efficient provision of a first fluid for forming the pinned droplets 71, 72, 73. The pinned droplets can then be separated by a non-conductive second fluid. In other words, the second side of the substrate with the sub-volumes for the pinned droplets can be transiently fluidically coupled in a common volume or common space, wherein in a following step individual separated droplets are formed that are electrically isolated by a further non-conductive fluid. However, there is no need for mechanical boundaries or sidewalls for electrical decoupling. In the shown example, the fluidic channel 51 connects at least some of the pores 20 provided on the second side of the substrate. A single channel may be provided covering all pores. However, it is also possible to provide multiple channels each covering a subset of pores.

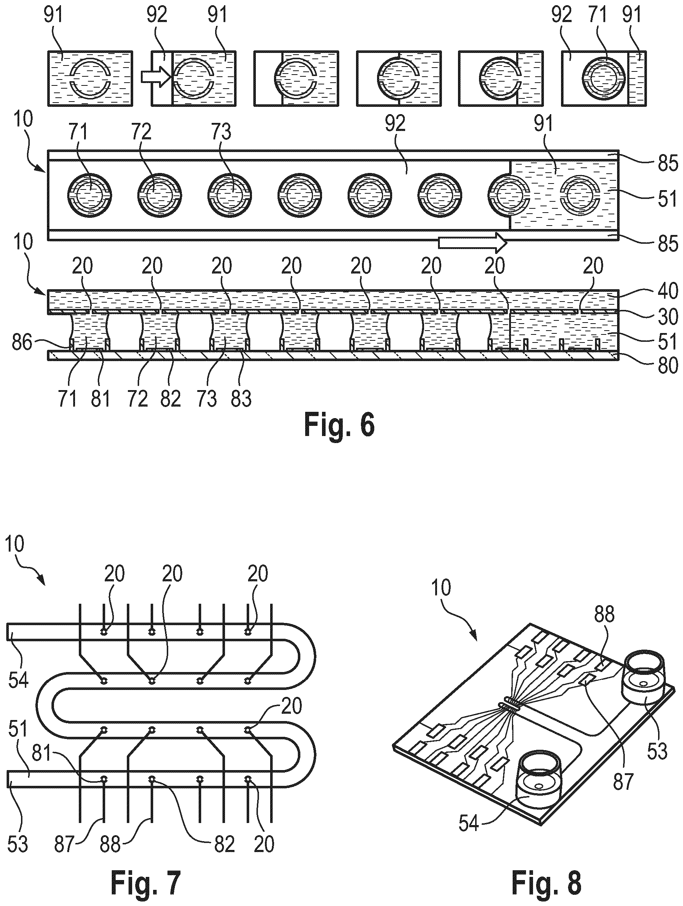

The device 10 is configured for providing the pinned droplets 71, 72, 73 by flushing the second side of the substrate 30 with a first liquid, wherein part of the first liquid is retained at the nanopores 20 for formation of the pinned droplets 71, 72, 73, and flushing the second side of the substrate 30 with a second fluid different from the first liquid for removing the first liquid between the pinned droplets of the first liquid. Accordingly, the pinned droplets remain only at the nanopores 20 and a space between is free from said liquid forming the pinned droplets. For example, the first liquid can be conductive or adapted for performing a nanopore measurement and the second fluid can be non-conductive. This will be described in more detail with reference to Fig. 6.

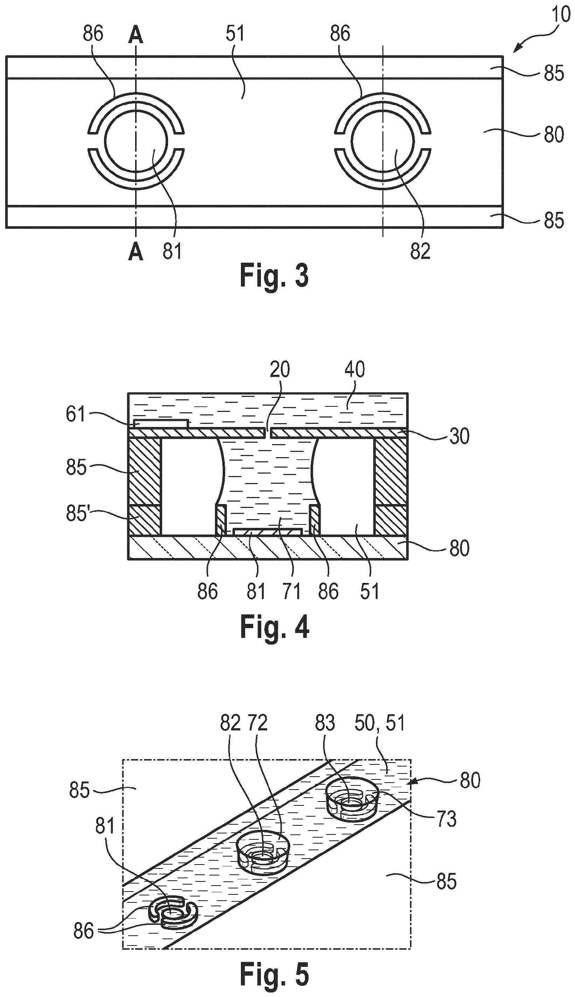

Fig. 3 to Fig. 5 illustrate a further exemplary embodiment of a nanopore (array) device 10. Fig. 3 shows a top view on a carrier 80 with confinement structures 86. Fig. 4 shows a schematic cross-sectional view of the nanopore array device 10 of Fig. 3 along line A-A. Fig. 5 shows a schematic isometric view of the nanopore array device 10 of Fig. 3 and 4.

The nanopore array device 10 comprises a substrate 30 with a plurality of pores 20 in said substrate. Fig. 4 shows a cross-sectional view of the nanopore array device 10 in a direction transverse to a longitudinal direction of a fluidic channel 51. A first fluid compartment 40 is arranged on a first side of the substrate 30, here on the top side. The first fluid compartment 40 is again fluidically coupled to the plurality of pores 20 from the first side. A first electrode 61 is adapted to be electrically coupled to the first fluid compartment 40. On a second side of the substrate 30 opposite to the first side of the substrate, a fluidic channel 51 is provided. The fluidic channel 51 is provided between the substate 30 and a carrier 80. Side walls 85, also referred to as channel walls, can provide a lateral boundary of the fluidic channel 51. The side walls 85 can be fabricated in a microfabrication process. For example, the side walls 85 can be fabricated in a lithographic process and may for example be made from a photoresist or by etching into the carrier 80. It will be understood that the choice of material is not limited thereto and may depend on the substances to be analyzed.

For formation of the pinned droplets 71, 72,73 at the respective nanopores 20 at each of said nanopores there is provided a confinement structure at the second side of the substrate 30. In the example shown in Fig. 3 to Fig. 5, the confinement structure comprises one or more barriers 86 adapted to retain a droplet directly below and aligned with a corresponding nanopore 20. The confinement structure or a part thereof can be provided on the second side of the substrate 30. As an alternative or in addition, the confinement structure or a part thereof can be arranged on the optional carrier 80.

Advantageously, a height of the barrier 86 is less than a height of a separation between the substrate 30 and the carrier 80. In particular, the height of the barrier 86 is less than a height of a fluidic channel 51. The barrier 86 can for example be made from a photoresist. In particular, a same photoresist that can also be used for formation of at least a portion 85' of the sidewalls 85 can be used.

In the example shown in Fig. 3 to 5 and as illustrated in the top view in Fig. 3, the barriers 86 can have a shape of an arc. In a refinement, the barriers 86 can have openings in an upstream direction and/or in a downstream direction in a longitudinal direction of the fluidic channel 51.

The confinement structure is adapted to capture and retain a droplet 71 from a fluid passing said confinement structure at the second side of the substrate 30. One or more barriers 86 can be adapted accordingly. However, also other confinement structures can be adapted accordingly, in particular by providing a region having a different wettability and/or surface energy and configured such that a pinned droplet is formed and retained at said region having the different wettability and/or surface energy from a fluid passing said confinement structure at the second side of the substrate 30.

Fig. 5 shows an isometric view with two of the droplets 72, 73 being formed at respective barriers 86. An array of second electrodes 81, 82, 83 is adapted to be electrically coupled to the pinned droplets 72, 73 at, in particular directly below and aligned with, at least some of the plurality of pores. Note that the substrate 30 with the pores is not shown in the partial perspective view in Fig. 5.

Referring now to Fig. 6, an exemplary process of forming a plurality of respective pinned droplets at at least some of a plurality of pores, preferably at all pores, at a second side of the substrate is shown. The nanopore device 10 can be configured as described in the present disclosure, for example in Fig. 2 or in Fig. 3 to Fig. 5. Note that the nanopore device 10 can be a non-prefilled device. The nanopore device 10 can thus be used with different liquids depending on the desired application. Thereby the nanopore device 10 can be suitable for many different measurement applications.

The first row in Fig. 6 shows a sequence of images illustrating the process of flushing a nanopore array device with a first liquid 91 and second fluid 92 for formation of pinned droplets.

The second row in Fig. 6 illustrates a top view of a row of pinned droplets 71, 72, 73 being formed. The third row in Fig. 6 illustrates a side view of a row of pinned droplets 71, 72, 73 each being formed at a corresponding pore 20 in the substrate 30.

As indicated by the arrows, the nanopore array device 10 is configured for providing the pinned droplets 71, 72, 73 by flushing the second side of the substrate 30 with a first liquid 91, whereby part of the first liquid 91 is retained at the pores 20, in particular at confinement structures at, in particular aligned with the respective pores at the second side of the substrate, for formation of the pinned droplets 71, 72, 73, and for flushing the second side of the substrate 30 with a second fluid 92 different from the first liquid 91 for removing the first liquid 91 between the pinned droplets 71, 72, 73 of the first liquid 91. The second fluid 92 can be an electrically insulating fluid including but not limited to air or oil. Generally speaking, the nanopore array device 10 can be adapted to electrically isolate neighboring droplets due to separation by an electrically insulating fluid. Hence, instead of providing wall members or partitions between neighboring (sub-)compartments, neighboring droplets may be isolated from one another by an electrically insulating fluid only. This can significantly reduce manufacturing complexity and overcome the need for complex microstructures for forming closed compartments or for electrical insulation.

Fig. 7 shows a schematic top view of a further exemplary embodiment of a nanopore array device 10. An array of nanopores 20 is provided. A pitch between neighboring pores 20 can for example be between 50 µm and 1.5 mm, in particular between 100 µm and 1 mm, in particular between 0.2 mm and 0.7 mm. Here a two-dimensional array of 4x4 nanopores 20 is provided. However, it is also possible to provide other two-dimensional arrays or also 1-dimensional arrays or line arrays. Accordingly, instead of providing a line of pinned droplets for a respective line of respective pores as shown in Fig. 6, also a two-dimensional array may be provided.

Instead of providing a separate fluidic channel for each individual nanopore, a shared fluidic channel 51 connecting at least some of the pores is provided. The fluidic channel can have an inlet 53 and an outlet 54.

A corresponding array of second electrodes adapted to be electrically coupled to the pinned droplets to be formed at at least some, preferably at all of the nanopores can also be provided for a two-dimensional array as exemplarily illustrated in Fig. 7 with electrodes 81 and 82 as well as the corresponding electrical connections 87 and 88. Such electrical connections can be provided with very small area requirement by known semiconductor fabrication, microelectrode array fabrication or PCB (printed circuit board) fabrication techniques.

Fig. 8 shows a line rendering of a photograph of an embodiment of a nanopore array device. The corresponding inlet 53 and outlet 54 for a fluid channel connecting at least some of the pores can be seen. Moreover, the respective electrical connections 87 and 88 for contacting second electrodes are shown.

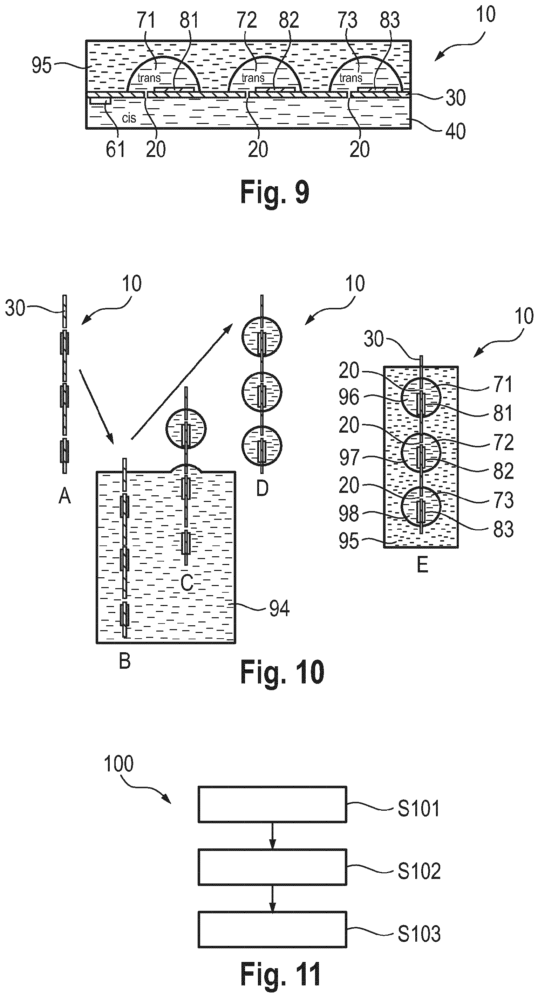

Fig. 9 shows a schematic cross-sectional view of a further exemplary embodiment of a nanopore device 10, here as a nanopore array device. The general operation principle is similar to the above. In the following, some differences over the previous embodiments are highlighted. The nanopore array device 10 can comprise a more open layout. Instead of providing an additional carrier 80 for forming a liquid channel between the carrier 80 and the substrate 30, it can be sufficient to provide a substrate 30 with the nanopores 20 in said substrate. The substrate 30 can be adapted for formation of a respective pinned droplet 71, 72, 73 at one or more, in particular at at least some of said plurality of pores 20 at a second side of the substrate 30. The one or more pinned droplets may be covered by a substance 95, for example by oil to prevent or reduce evaporation. This allows to provide a device that can be manufactured at low cost. Moreover the second side of the membrane 30 for formation of the pinned droplets is easily accessible. The pinned droplets can form the respective *trans* sub-compartments. Instead of providing complex liquid channel connections, it may be sufficient to simply dip the nanopore array device is a first fluid for formation of the pinned droplets 71, 72, 73. In an optional further step, the pinned droplets may be immersed e.g. into oil to cover them. The first compartment 40 for the nanopore measurement may be provided as a shared compartment on the opposite first side of the substrate 30.

A first electrode 61 is again adapted to be electrically coupled to the first fluid compartment. For example, an electrode 61 may be provided on the first side of the substrate, here on the bottom side. An array of second electrodes 81, 82, 83 is adapted to be electrically coupled to the pinned droplets 71, 72, 73 at at least some of said plurality of pores 20 of the nanopore array device 10.

Fig. 10 shows the process of forming droplets on both sides of a further exemplary nanopore array device 10. In a further refinement, the nanopore array device 10 can be further configured for formation of a pinned droplet 96, 97,98 at at least some of said plurality of pores 20 at the first side of the substrate 30. Similar to the second side, also on the first side at each of said pores there can be provided a further confinement structure for formation of the pinned droplets at the respective pores. Instead of providing a shared first compartment, it is thus also possible to provide the first compartment sub-divided into sub-compartments in form of pinned droplets 96, 97, 98.

In Fig. 10, the sequence is exemplarily indicated by capital letters A to E. The respective nanopore array device 10 is provided in step A. The nanopore array device is then immersed in a first liquid 94 such as salt water or the like adapted for nanopore measurement. The nanopore array device 10 is then retracted as shown by steps C and D. As shown in step D, the nanopore array device 10 is thus provided with a double-sided pinned droplet array. An array of first electrodes is adapted to be electrically coupled to the pinned droplets at at least some of the plurality of pores on the first side of the substrate 30. An array of second electrodes is adapted to be electrically coupled to the pinned droplets at at least some of the plurality of pores on the second side. An advantage can be that a large number of *cis* and *trans* sub-compartments for a nanopore measurement can be provided very efficiently. In an optional step E, the nanopore array device may be immersed in a further liquid 96, in particular a non-conductive liquid such as oil, to reduce evaporation.

In at least some of the embodiments described above, at least some of the pores 20 can be chemically or biologically functionalized. In particular, at least some of the pores 20 can host a lipid bilayer, a polymer membrane, and/or a biological protein.

Fig. 11 shows a flow chart of a method 100 for operating a nanopore array device. In a first step S101, a nanopore array device as disclosed herein is provided. In a second step S102, the second side of the substrate is flushed with a first liquid, wherein part of the first liquid is retained at the pores for formation of the pinned droplets. In a third step S103, the second side of the substrate is flushed with a second fluid different from the first liquid for removing the first liquid between the pinned droplets of the first liquid. It shall be understood the additional steps may be provided before or after the above-mentioned steps. For example, the first fluid compartment may be filled and/or an analyte may be provided.

In an optional further refinement, the method can comprise optional further steps. For example, in a fourth step, the second side of the substrate can be flushed with a third fluid different from the first and/or second fluid. The third fluid can be adapted to remove the first fluid previously retained at the pores. The third fluid can be a third liquid. Part of the third liquid can be retained at the pores for formation of the pinned droplets of the third liquid. In a fifth step, the second side of the substrate can be flushed with a fourth fluid different from the third liquid for removing the third liquid between the pinned droplets of the third liquid. Hence, the respective fluids and liquids can be easily exchanged. A further advantage can be a rapid solution exchange.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Nanopore device (10), in particular a nanopore array device, comprising
- a substrate (30);
- one or more nanopores (20) in said substrate;
- a first fluid compartment (40) arranged on a first side of the substrate, wherein the first fluid compartment is fluidically coupled to the one or more nanopores (20) from the first side;
- a first electrode (61) adapted to be electrically coupled to the first fluid compartment (40);
wherein the nanopore device (10) is configured for formation of a pinned droplet (71, 72, 73) at at least one of said one or more nanopores (20) at a second side of the substrate (30), wherein the second side of the substrate is opposite to the first side of the substrate; and
one or more second electrodes (81, 82, 83) adapted to be electrically coupled to the one or more pinned droplets (71, 72, 73) at said one or more nanopores (20).

2. Nanopore device according to claim 1, wherein the nanopore device (10) is a nanopore array device, comprising
a plurality of nanopores (20) in said substrate (30);
wherein the first fluid compartment (40) is fluidically coupled to the plurality of nanopores (20) from the first side;
wherein the nanopore array device is configured for formation of a pinned droplet (71, 72, 73) at at least some of said plurality of nanopores (20) at the second side of the substrate (30); and
an array of second electrodes (81, 82, 83) adapted to be electrically coupled to the pinned droplets (71, 72, 73) at at least some of said plurality of nanopores (20).

3. Nanopore device according to any of the preceding claims, wherein for formation of the one or more pinned droplets (71, 72,73) at the respective one or more nanopores (20), at each of said one or more nanopores there is provided a confinement structure on the second side of the substrate (30).

4. Nanopore device according to claim 3, wherein the confinement structure is adapted to capture and retain a droplet (71, 72,73) from a fluid (91) passing said confinement structure at the second side of the substrate (30).

5. Nanopore device according to claim 3 or **4,** wherein the confinement structure is provided by a region having a different wettability and/or surface energy and configured such that a pinned droplet is formed and retained at said region having the different wettability and/or surface energy.

6. Nanopore device according to any of the preceding claims, wherein (a) said one or more pinned droplets (71, 72,73) each having a fluid volume of at most 100 nL, in particular having a fluid volume between 5 pL and 100 nL, in particular between 7 pL and 50 nL, in particular between 10 pL and 1 nL; and/or (b) a pitch between neighboring nanopores (20) is between 50 µm and 1.5 mm, in particular between 100 µm and 1 mm, in particular between 0.2 mm and 0.7 mm.

7. Nanopore device according to any of the preceding claims, wherein at least two nanopores (20) are fluidically coupled on the second side of the substrate (30); in particular wherein the nanopores (20) are fluidically coupled on the second side of the substrate (30) where the nanopore array device (10) is configured for formation of the pinned droplets (71, 72, 73).

8. Nanopore device according to claim 7, wherein a fluidic channel (51) connecting at least some of the nanopores (20) is provided on the second side of the substrate (30).

9. Nanopore device according to any of the proceeding claims, wherein the nanopore device (10) is configured for providing the one or more pinned droplets (71, 72, 73) by flushing the second side of the substrate (30) with a first liquid (91), wherein part of the first liquid (91) is retained at the one or more nanopores (20) for formation of the pinned droplets (71, 72, 73), and flushing the second side of the substrate (30) with a second fluid (92) different from the first liquid (91) for removing the first liquid (91) from around and/or between the one or more pinned droplets (71, 72, 73) of the first liquid (91).

10. Nanopore device according to any of the preceding claims, wherein the nanopore device (10) is further configured for formation of a pinned droplet (96, 97, 98) at said one or more nanopores (20) at the first side of the substrate (30).

11. Nanopore device according to any of the preceding claims, wherein one or more nanopores (20) are chemically or biologically functionalized, in particular wherein one or more nanopores is configured to host a lipid bilayer membrane, polymer membrane, and/or a biological protein.

12. Nanopore device according to any of the preceding claims, wherein the nanopore device (10) is a non-prefilled device.

13. Nanopore device according to any of the preceding claims, wherein the nanopore device (10) is adapted to electrically isolate neighboring droplets (71, 72, 73) due to separation by an electrically insulating fluid (92).

14. System (11) for performing nanopore based analysis; the system comprising:
- a nanopore device (10) according to any of the preceding claims; and an
- an analyzer (60), wherein the analyzer is adapted to detect and determine physicochemical properties of one or more molecules (2), in particular of biological or synthetic polymers, in particular one or more of their size, shape and sequence, based on a current and/or voltage change over time across one or more nanopores (20) in said substrate (30) acquired via the first electrode (61) and the one or more second electrodes (81, 82, 83).

15. Method (100) for operating a nanopore device, in particular a nanopore array device, the method comprising the steps of:
- providing a nanopore device according to any of the preceding claims (S101);
- flushing the second side of the substrate with a first liquid, wherein part of the first liquid is retained between the one or more nanopores and the one or more second electrodes for formation of the one or more pinned droplets (S102); and
- flushing the second side of the substrate with a second fluid different from the first liquid for removing the first liquid around and/or between the one or more pinned droplets of the first liquid (S103).
